(19) Europäisches Patentamt
European Patent Office
Office européen des brevets

(11) Publication number: **0 262 710 B1**

# (12) EUROPEAN PATENT SPECIFICATION

(45) Date of publication of patent specification: **12.08.92**

(51) Int. Cl.[5]: **A61K 37/02**, A61K 39/02, G01N 33/564, G01N 33/68, C07K 13/00, C07K 15/00, C12N 15/00, C12N 1/20, C12N 7/00, //(C12N1/20, C12R1:42)

(21) Application number: **87201691.0**

(22) Date of filing: **07.09.87**

## (54) Use of a peptide for the preparation of compositions for the alleviation, treatment and diagnosis of autoimmune diseases, especially arthritic conditions, compounds related to said peptide, micro-organisms expressing said peptide or a compound related to said peptide, as well as pharmaceutical and diagnostic compositions and test kits.

(30) Priority: **09.09.86 NL 8602270**
**14.05.87 NL 8701163**

(43) Date of publication of application:
**06.04.88 Bulletin 88/14**

(45) Publication of the grant of the patent:
**12.08.92 Bulletin 92/33**

(84) Designated Contracting States:
**AT BE CH DE ES FR GB GR IT LI LU NL SE**

(56) References cited:
**WO-A-85/05034**

(73) Proprietor: **DE STAAT DER NEDERLANDEN VERTEGENWOORDIGD DOOR DE MINISTER VAN WELZIJN, VOLKSGEZONDHEID EN CULTUUR**
**P.O. Box 439**
**NL-2260 AK Leidschendam(NL)**

Proprietor: **Rijksuniversiteit Utrecht**
**Heidelberglaan 8**
**NL-3584 CS Utrecht(NL)**

Proprietor: **YEDA RESEARCH AND DEVELOPMENT COMPANY LIMITED**
**P.O. Box 95**
**Rehovot 76100(IL)**

EP 0 262 710 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**INFECTION AND IMMUNITY, vol. 50, no. 3, December 1985, pages 800-806, American Society for Microbiology; J.E.R. THOLE et al.: "Cloning of Myocobacterium bovis BCG DNA and expression of antigens in Escherichia coli"**

**BIOLOGICAL ABSTRACTS, vol. 82, no. 2, 1986, page AB-444, abstract no. 13678, Biological Abstracts, Inc., Philadelphia, PA., US; F. EMMRICH et al.: "A recombinant 64 kilodalton protein of Mycobacterium bovis BCG specifically stimulates human T4 clones reactive to mycobacterial antigens", & J. EXP. MED. 163(4), 1024-1029, 1986**

**THE LANCET, vol. 2, no. 8502, 9th August 1986, pages 310-313, London, GB; T.H.M. OTTENHOFF et al.: "Evidence for an HLA-DR4-associated immune-response gene for mycobacterium tuberculosis"**

**PROCEEDINGS OF THE NATIONAL ACADEMY OF SCIENCE OF THE USA, vol. 82. May 1985, pages 2583-2587, Washington, D.C., US; R.A. YOUNG et al.: "Dissection of Mycobacterium tuberculosis antigens using recombinant DNA"**

**INFECTION IMMUNITY, vol. 55, no. 6, June 1987, pages 1466-1475, American Society for Microbiology; J.E.R. THOLE et al.: "Characterization, sequence determination, and immunogenicity of a 64-kilodalton protein of Mycobacterium bovis BCG expressed in Escherichia coli K-12"**

**INFECTION AND IMMUNITY, vol. 55, no. 8, August 1987, pages 1932-1935, American Society for Microbiology; T.M. SHINNICK et al.: "The etiologic agents of leprosy and tuberculosis share an immunoreactive protein antigen with the vaccine strain Mycobacterium bovis BCG"**

**J. Exp. Med. 1986, 163(4), 1024-1029**

(72) Inventor: **Van Eden, Willem**
**Soestdijkseweg 399,**
**NL-3723 HD Bilthoven,(NL)**
Inventor: **Thole, Jelle Egbertus Rudolfus,**
**Albert Loethoelistraat 216,**
**NL-1111 KZ Diemen,(NL)**
Inventor: **Van Embden, Johannes Dirk Anthonie,**
**Van Limburg Stirumstraat 15,**
**NL-3781 VB Utrecht,(NL)**
Inventor: **Van der Zee, Ruurd,**
**Radijsstraat 17,**
**NL-9741 BJ Groningen,(NL)**
Inventor: **Cohen, Irun Robert,**
**11, Hankin Street,**
**Rehovot,(IL)**

(74) Representative: **van der Beek, George Frans, Ir.**
**Nederlandsch Octrooibureau Scheveningseweg 82 P.O. Box 29720**
**NL-2502 LS 's-Gravenhage(NL)**

## Description

The present invention relates to the use of a certain peptide for the preparation of compositions for the alleviation, treatment and diagnosis of autoimmune diseases, especially arthritic conditions. The invention further relates to compounds related to said peptide, to micro-organisms expressing said related compounds, and to pharmaceutical and diagnostic compositions comprising a compound related to said peptide, and to test kits for performing immunological tests.

### Background of the invention

Millions of persons are afflicted with chronic forms of arthritis which are thought to involve autoimmunity to constituents of the joints or connecting tissues of the body. These conditions inlcude rheumatoid arthritis, ankylosing spondylitis, Reiter's syndrome and other forms of reactive arthritis. The etiology of these diseases is not known, but previous infection with various microbes seems to act as an inciting circumstance in genetically susceptible individuals. For example, patients with rheumatoid arthritis may show unusual reactivity to mycobacterial intigens and immunization with the BCG strain of mycobacteria was found to lead to arthritis in 15 of 150 individuals. Ankylosing spondylitis has been associated with infection by Klebsiella or Yersinia species of bacteria and other cases of arthritis by Salmonella, Shigella, etc. There is no evidence of active infection of joints by these microbes in the vast majority of cases and it has been postulated that microbial infection may trigger an aberrant, autoimmune response of the individual against his own antigens present in the joints. Adjuvant arthritis (AA) is an experimental model of arthritis inducible by immunizing susceptible strains of rats to Mycobacteria. The disease which develops about 12 days after immunization has many of the features of rheumatoid arthritis and AA has been considered to be a model of rheumatoid arthritis.

### Prior art.

EP A O 181 364 discloses aqueous acetone soluble and insoluble fractions of certain mycobacteria, such as Mycobacterium H-37, M. kansasii and M. vaccae. The soluble fraction of Myc. H-37 was found to provoke an immune response leading to resistance to adjuvant arthritis. The insoluble fraction seemed to be responsible for induction of adjuvant arthritis. Micobacterium vaccae was shown to be substantially free of adjuvant arthritis inducing components. Further, EP A O 181 364 describes certain lines and clones of T-lymphocytes selected for their reactivity to mycobacteria. These can be used for producing arthritis upon inoculation into irradiated rats. One line, designated as A2 was found to induce arthritis upon intravenous injection into irradiated rats. The same line, A2 is effective in vaccinating unirradiated rats against subsequent autoimmune arthritis induced by active immunization to mycobacteria. Cell line A2 has been cloned. There were obtained two distinct clones, designated as A2b and A2C, respectively. A2b causes arthritis but does not vaccinate against it; clone A2c does not cause arthritis but vaccinates against it. In addition to preventing arthritis, clone A2c can be used to treat AA. Moreover, clones A2b and A2c can be used to identify antigens associated with arthritogenicity or with suppression of arthritogenicity. Both clones respond to whole mycobacteria as well as to cartilage proteoglycan.

### Description of the invention

According to the present invention it was found that a° polypeptide having a molecular mass of about 64 kD, the preparation of which is described in Infection and Immunity 1985, pages 800-806, is useful as an immunogen inducing resistance to autoimmune arthritis and similar autoimmune diseases.

In the above-mentioned article the peptide in question is called Antigen A and this designation will be used here as well. Antigen A was obtained by constructing a gene bank of Mycobacterium bovis BCG DNA in Escherichia coli by cloning Sau3A-cleaved mycobacterium DNA fragments into the lambda vector EMBL3. The expression of mycobacterial antigens was analyzed by Western blotting with hyperimmune rabbit sera. The article states that among 770 clones tested, several were found that produced various mycobacterial antigens in low amounts, with concentrations generally close to the detection limit. One particular clone was chosen for further investigation. This clone produced a 64 kD antigen. By placing the lambda promoter $P_L$ in front of the structural gene of this antigen, an overproducing E. coli strain was obtained. The article shows that antigens cross-reacting with the 64 kD protein are present in a wide variety of mycobacteria and also in so-called purified protein derivatives which are routinely used for skin tests. Finally, it is stated in the article that preliminary experiments indicate the presence of antibodies against the

64 kD antigen in sera from tubercolosis patients.

Antigen A was found to have the following amino acid sequence:

```
  1  MAKTIAYDEE ARRGLERGLN ALADAVKVTL GPKGRNVVLE KKWGAPTITN DGVSIAKEIE
 61  LEDPYEKIGA ELVKEVAKKT DDVAGDGTTT ATVLAQALVR EGLRNVAAGA NPLGLKRGIE
121  KAVEKVTETL LKGAKEVETK EQIAATAAIS AGDQSIGDLI AEAMDKVGNE GVITVEESNT
181  FGLQLELTEG MRFDKGYISG YFVTDPERQE AVLEDPYILL VSSKVSTVKD LLPLLEKVIG
241  AGKPLLIIAE DVEGEALSTL VVNKIRGTFK SVAVKAPGFG DRRKAMLQDM AILTGGQVIS
301  EEVGLTLENA DLSLLGKARK VVVTKDETTI VEGAGDTDAI AGRVAQIRQE IENSDSDYDR
361  EKLQERLAKL AGGVAVIKAG AATEVELKER KHRIEDAVRN AKAAVEEGIV AGGGVTLLQA
421  APTLDELKLE GDEATGANIV KVALEAPLKQ IAFNSGLEPG VVAEKVRNLP AGHGLNAQTG
481  VYEDLLAAGV ADPVKVTRSA LQNAASIAGL FLTTEAVVAD KPEKEKASVP GGGDMGGMDF
```

Detailed discussion of the invention.

As mentioned above clones A2b and A2c as disclosed in °EP A O 181 364 can be used to identify antigens associated with arthritogenicity or with suppression of arthritogenicity. Both clones respond to whole mycobacterial and both A2b and A2c respond to antigen A.

T-cell clones A2b, and A2c and control cell-line Cla (anti-ovalbumin) were assayed for in vitro proliferative responses to Micobacterium tuberculosis, Antigen A, E. coli control lysate, ovalbumin (OVA) and mitogen ConA in a standard test (20 x $10^3$ clone/line cells, 2 x $10^6$ irradiated accessory cells and antigens in optimum concentration per well, $^3$H-Thymidine incorporation for 18 hours after 48 hours of incubation). The following table A shows the test results which are expressed as stimulation indexes.

TABLE A.

| | M. tub. | Ant. A | coli contr. | OVA | ConA |
|---|---|---|---|---|---|
| A2b | 180 | 500 | 2.9 | - | 430 |
| A2c | 304 | 516 | 1.5 | - | 390 |
| Cla | - | 1.5 | 1.2 | 45 | 64 |

The in vivo potency of Antigen A was checked by immunizing rats with Antigen A before and after induction of arthritis with M. tuberculosis. The test with challenge after immunization was carried out as follows:

Groups of 4 Lewis rats wer treated by intraperitoneal inoculation of water, Antigen A (50 μg) and E. coli control lysate (amount equivalent to coli content of 50 μg Antigen A) in oil. 35 Days later, susceptibility to induction of adjuvant arthritis was tested by inoculating the rats intracutaneously with M. tuberculosis (1 mg) in oil. Occurrence of arthritis was checked by daily inspection of the rat joints. The results are shown in table B.

TABLE B.

| Primary immunization | | Secondary challenge (35 days later) with M. tuberculosis in oil. | |
|---|---|---|---|
| Inoculum in oil | Arthritis incidence | Arthritis incidence | Clinical grade |
| Water | 0/4 | 4/4 | severe |
| Antigen A | 0/4 | 2/4 | very mild |
| E. coli contr. | 0/4 | 4/4 | severe |

The tests involving inoculation after induction of autoimmune arthritis were carried out as follows:

Arthritis was induced by inoculating groups of 3 Lewis rats with M. tuberculosis (1 mg) in oil intracutaneously. 3 Days later the rats were treated by intraperitoneal inoculation of water, Antigen A (200 µg) and E. coli control lysate (amount equivalent to coli content of 200 µg Antigen A) in oil. Ocurrence of arthritis was checked by daily inspection of the rat joints.

The results are shown in table C.

TABLE C.

| Inoculum administered at day 3 after disease induction | Arthritis | |
|---|---|---|
| | incidence | clinical grade |
| Water | 3/3 | severe |
| Antigen A | 1/3 | very mild |
| E. coli contr. | 3/3 | severe |

It is seen that Antigen A is not arthritogenic by itself but reduces the incidence of arthritis after active induction disease with 50%, and also reduces the severity of remaining disease remarkably. A similar reduction of disease incidence and severity is seen when Antigen A is administered three days after disease is induced. E. coli itself has no effect. Thus, Antigen A is arthritis suppressive, while not being arthritogenic.

Further, it was found that Antigen A cross-reacts with similar proteins present in various other mycobacteria and E. coli and with Treponema and gram-negative enterobacteria. This cross-reactivity is shown in the following table D.

TABLE D.

Cross-reactivity between Antigen A and antigens present in other bacteria.

| | Antig.A | 64kD of mycobact. | E.coli 60kD | Trep.poll | Shig. | Salmon. | Klebsiella |
|---|---|---|---|---|---|---|---|
| MCA HATR 1-24 | + | - | + | + | + | + | + |
| F47-10 | + | + | + | | + | + | + |
| Polycl.anti comm.ag. Legion/ Pseudom. | + | + | + | + | + | + | + |

Serological cross-reactivity as shown by Western-blot analysis. HATR 1-24 and F47-10 are monoclonal antibodies raised against Treponema and Mycobacterium tuberculosis respectively.

The polyclonal serum was raised against the common antigen of Legionella and Pseudomonas.

This indicates that epitopes present on Antigen A are similarly present on presumably equivalent proteins of various bacterium species, such as from Mycobacterium, Escherichia, Treponema, Shigella, Salmonella, Yersinia, Nocardia, Campylobacter, or Klebsiella species. Particularly, antigen A amino acid sequence 190-213 is also present in a corresponding 65 KD protein from Mycobacterium leprae, with the exception that, in the M. leprae protein, amino acid 206 is not proline, but alanine.

Further, it was found that only part of the Antigen A sequence is responsible for the stimulating activity upon T-cell clones A2b and A2c. This was determined by testing Antigen A fragments, namely truncated derivatives produced by deletion mutants of the gene, fusion proteins with $\beta$-galactosidase and proteolysis products of Antigen A, for their ability to stimulate said T-cell clones. These fragments were obtained by means of recombination-DNA techniques, by incorporating parts of the Antigen A gene, in some cases fused to the $\beta$-galactosidase gene, into a plasmide and expressing in E. coli K12 M1070.

The peptide with Antigen A amino acid sequence 234-540 was shown not to stimulate clones A2b and A2c. However, the fragment lacking amino acid sequence 481-540 did. $\beta$-Galactosidase-fused peptides with Antigen A amino acid sequences 61-540, 109-540 and 171-540 were reactive, those with amino acid sequences 272-540 and 280-540 were not reactive. $\beta$-Galactosidase alone was not reactive.

Therefore, the epitope responsible for the stimulation of T-cell clones A2b and A2c resides in amino acid sequence 171-234.

Further, it was found that a synthetic peptide having Antigen A amino acid sequence 180-196 is also recognized by T-cell clones A2b and A2c. Therefore, any polypeptide composed of at least 17 amino acid residues and comprising in its molecule Antigen A amino acid sequence 180-196 will contain the epitope responsible for stimulation of T-cell clones A2b and A2c. An example of such a polypeptide is the polypeptide having Antigen A amino acid sequence 171-240.

Consequently, the invention relates to the use of Antigen A, that is, the peptide having the sequence of 540 amino acids mentioned earlier in this specification, for the preparation of compositions for the alleviation, treatment and diagnosis of autoimmune diseases, especially arthritic conditions.

The invention also relates to polypeptides composed of 17 to 70 amino acid residues and comprising in their molecule Antigen A amino acid sequence 180-196.

More specifically, the invention relates to the polypeptide having Antigen A amino acid sequence 171-240 which is

```
171        181        191        201
 GVITVEESNT FGLQLELTEG MRFDKGYISG YFVTDPERQE

211        221        231
 AVLEDPYILL VSSKVSTVKD LLPLLEKVIG.
```

Although T-cell clones A2b and A2c respond to all of the above-defined polypeptides, the antigenicity and immunogenicity of the polypeptides may be enhanced by coupling thereto at least one radical capable of improving the presentation of the antigenic determinants of the polypeptides. Such radicals are known in the art, and comprise, for example, radicals of peptides, tetanus toxoid, diphtheria toxoid, $\beta$-galactosidase, and microbial outer membrane proteins. Multimers of the polypeptides in question are also contemplated. These modified polypeptides also form part of the invention.

All of the polypeptides of the invention, namely the polypeptides composed of 17-70 amino acid residues and comprising in their molecule Antigen A amino acid sequence 180-196, the above defined modified peptides including the multimers, can be used as immunogens in pharmaceutical compositions, especially vaccines for the alleviation and treatment of autoimmune diseases, especially arthritic conditions, and also as antigens in diagnostic compositions for the diagnosis of these diseases. These pharmaceutical and diagnostic compositions, which may be prepared in a way known in the art, also form part of the invention.

Another way to improve the immunogenicity of the polypeptides according to the invention is to construct, by known genetical engineering methods, microorganisms expressing a polypeptide according to the invention either as such or as part of a fusion protein or as a multimer thereof. These microorganisms can be used for the preparation of a live vaccine which will provoke not only the production of antibodies against the micro-organism in question, but will also be useful for the alleviation and treatment of autoimmune diseases. These genetically engineered microorganisms, and pharmaceutical compositions containing these, also form part of the invention. Examples of suitable genetically engineered microorganisms are Vaccinia and Salmonella strains.

Finally, the invention provides kits for performing immunological tests comprising a container with at least one of the antigenic compounds discussed above, or a container with the diagnostic composition mentioned above.

The antigenic compounds and diagnostic compositions as well as the diagnostic kits according to the invention may be used for various types of assays, such as:

a.1. a lymphocyte proliferation test, or determination of any entity indicative of such proliferation;

a.2. indicative of the measure of lymphocyte activation are also changes which can be assayed by standard means so as to establish the presence and degree of lymphocyte activation: amongst these there may be mentioned:

- a. production of lymphokines (such as interleukin-2 (IL-2));
- b. gamma interferon;
- c. migration inhibition factor (MIF);
- d. expression of membrane markers, such as IL-2 receptor; peanut agglutination receptor;
- e. expression of enzymes such as heparanase.

b. determination of antibody titer in absolute terms or as a ratio of the values obtained by different compositions, said values or ratios being indicative of the presence or absence of the disease. Quantitative values obtained are of use in establishing the severity of the disease.

The diagnostic compositions according to the invention may be prepared by combining one or more antigenic compounds according to the invention as above-defined with suitable adjuvants and auxiliary components. Standerdized kits with reference and calibration means are of value in the rapid and convenient determination of arthritic disease and its stage and/or severity.

## Claims

**1.** Use of a peptide of the formula

```
  1 MAKTIAYDEE ARRGLERGLN ALADAVKVTL GPKGRNVVLE KKWGAPTITN DGVSIAKEIE
 61 LEDPYEKIGA ELVKEVAKKT DDVAGDGTTT ATVLAQALVR EGLRNVAAGA NPLGLKRGIE
121 KAVEKVTETL LKGAKEVETK EQIAATAAIS AGDQSIGDLI AEAMDKVGNE GVITVEESNT
181 FGLQLELTEG MRFDKGYISG YFVTDPERQE AVLEDPYILL VSSKVSTVKD LLPLLEKVIG
241 AGKPLLIIAE DVEGEALSTL VVNKIRGTFK SVAVKAPGFG DRRKAMLQDM AILTGGQVIS
301 EEVGLTLENA DLSLLGKARK VVVTKDETTI VEGAGDTDAI AGRVAQIRQE IENSDSDYDR
361 EKLQERLAKL AGGVAVIKAG AATEVELKER KHRIEDAVRN AKAAVEEGIV AGGGVTLLQA
421 APTLDELKLE GDEATGANIV KVALEAPLKQ IAFNSGLEPG VVAEKVRNLP AGHGLNAQTG
481 VYEDLLAAGV ADPVKVTRSA LQNAASIAGL FLTTEAVVAD KPEKEKASVP GGGDMGGMDF
```

for the preparation of compositions for the alleviation, treatment and diagnosis of autoimmune diseases, especially arthritic conditions.

**2.** A polypeptide useful for the diagnosis of, or as immunogen against autoimmune diseases, which polypeptide is composed of 17 to 70 amino acid residues and comprises in its molecule amino acid sequence 180-196 of the polypeptide mentioned in claim 1.

**3.** The polypeptide of claim 2 having the following amino acid sequence:

```
171          181          191          201
GVITVEESNT   FGLQLELTEG   MRFDKGYISG   YFVTDPERQE

211          221          231
AVLEDPYILL   VSSKVSTVKD   LLPLLEKVIG.
```

**4.** Compound according to claim 2 or 3 coupled to at least one radical enhancing its antigenicity and immunogenicity.

**5.** Compound according to claim 4 in which the radical enhancing the immunogenicity is a radical of a peptide, of tetanus toxoid, of diphtheria toxoid, of $\beta$-galactosidase, or of a microbial outer membrane protein.

**6.** Multimers of the compounds of claim 2 or 3.

**7.** Microorganisms expressing a compound of claim 2 or 3 either as such or as part of a fusion protein or as a multimer.

**8.** Microorganisms according to claim 7, which are genetically engineered Vaccinia or Salmonella strains.

**9.** A pharmaceutical composition, especially a vaccine against autoimmune diseases, in particular arthritic conditions comprising a compound of anyone of claims 2 to 6 or a microorganism according to claim 7 or 8.

**10.** A diagnostic composition comprising a compound of any one of claims 2 to 6.

**11.** A kit for performing immunological tests comprising a container with a compound of any one of claims 2 to 6 or with the diagnostic composition of claim 10.

**Revendications**

**1.** Utilisation d'un peptide de formule

1 MAKTIAYDEE ARRGLERGLN ALADAVKVTL GPKGRNVVLE KKWGAPTITN DGVSIAKEIE
61 LEDPYEKIGA ELVKEVAKKT DDVAGDGTTT ATVLAQALVR EGLRNVAAGA NPLGLKRGIE
121 KAVEKVTETL LKGAKEVETK EQIAATAAIS AGDQSIGDLI AEAMDKVGNE GVITVEESNT
181 FGLQLELTEG MRFDKGYISG YFVTDPERQE AVLEDPYILL VSSKVSTVKD LLPLLEKVIG
241 AGKPLLIIAE DVEGEALSTL VVNKIRGTFK SVAVKAPGFG DRRKAMLQDM AILTGGQVIS
301 EEVGLTLENA DLSLLGKARK VVVTKDETTI VEGAGDTDAI AGRVAQIRQE IENSDSDYDR
361 EKLQERLAKL AGGVAVIKAG AATEVELKER KHRIEDAVRN AKAAVEEGIV AGGGVTLLQA
421 APTLDELKLE GDEATGANIV KVALEAPLKQ IAFNSGLEPG VVAEKVRNLP AGHGLNAQTG
481 VYEDLLAAGV ADPVKVTRSA LQNAASIAGL FLTTEAVVAD KPEKEKASVP GGGDMGGMDF

pour la préparation de compositions pour l'atténuation, le traitement et le diagnostic de maladies auto-immunes, notamment d'affections arthritiques.

2. Polypeptide utile pour le diagnostic de, ou comme immunogène contre des, maladies auto-immunes, lequel polypeptide est formé de 17 à 70 résidus d'acides aminés et comprend dans sa molécule la séquence d'acides aminés 180-196 du polypeptide mentionné à la revendication 1.

3. Polypeptide selon la revendication 2, ayant la séquence d'acides aminés suivante :

171 GVITVEESNT 181 FGLQLELTEG 191 MRFDKGYISG 201 YFVTDPERQE
211 AVLEDPYILL 221 VSSKVSTVKD 231 LLPLLEKVIG.

4. Composé selon la revendication 2 ou 3 couplé à au moins un radical augmentant son antigénicité et son immunogénicité.

5. Composé selon la revendication 4, dans lequel le radical augmentant l'immunogénicité est un radical issu d'un peptide, de la toxine tétanique, de la toxine diphtérique, de la béta-galactosidase ou d'une protéine membranaire externe microbiologique.

6. Multimères des composés selon la revendication 2 ou 3.

7. Micro-organismes exprimant un composé selon la revendication 2 ou 3 en tant que tel ou comme partie d'une protéine fusion ou comme multimère.

8. Micro-organismes selon la revendication 7, qui sont des souches vaccine ou Salmonelle modifiées génétiquement.

9. Composition pharmaceutique, notamment vaccin contre des maladies auto-immunes, en particulier contre des affections arthritiques, comprenant un composé selon l'une quelconque des revendications 2 à 6 ou un micro-organisme selon la revendication 7 ou 8.

10. Composition de diagnostic comprenant un composé selon l'une quelconque des revendications 2 à 6.

11. Kit pour réaliser des tests immunologiques, comprenant un récipient contenant un composé selon l'une quelconque des revendications 2 à 6 ou la composition de diagnostic selon la revendication 10.

## Patentansprüche

1. Verwendung eines Peptids der Formel

| | | | | | | |
|---|---|---|---|---|---|---|
| 1 | MAKTIAYDEE | ARRGLERGLN | ALADAVKVTL | GPKGRNVVLE | KKWGAPTITN | DGVSIAKEIE |
| 61 | LEDPYEKIGA | ELVKEVAKKT | DDVAGDGTTT | ATVLAQALVR | EGLRNVAAGA | NPLGLKRGIE |
| 121 | KAVEKVTETL | LKGAKEVETK | EQIAATAAIS | AGDQSIGDLI | AEAMDKVGNE | GVITVEESNT |
| 181 | FGLQLELTEG | MRFDKGYISG | YFVTDPERQE | AVLEDPYILL | VSSKVSTVKD | LLPLLEKVIG |
| 241 | AGKPLLIIAE | DVEGEALSTL | VVNKIRGTFK | SVAVKAPGFG | DRRKAMLQDM | AILTGGQVIS |
| 301 | EEVGLTLENA | DLSLLGKARK | VVVTKDETTI | VEGAGDTDAI | AGRVAQIRQE | IENSDSDYDR |
| 361 | EKLQERLAKL | AGGVAVIKAG | AATEVELKER | KHRIEDAVRN | AKAAVEEGIV | AGGGVTLLQA |
| 421 | APTLDELKLE | GDEATGANIV | KVALEAPLKQ | IAFNSGLEPG | VVAEKVRNLP | AGHGLNAQTG |
| 481 | VYEDLLAAGV | ADPVKVTRSA | LQNAASIAGL | FLTTEAVVAD | KPEKEKASVP | GGGDMGGMDF |

zur Herstellung von Zusammensetzungen zur Linderung, Behandlung und Diagnose von Autoimmunkrankheiten, insbesondere arthritischen Zuständen.

**2.** Polypeptid, das für die Diagnose von oder als Immunogen gegenüber Autoimmunkrankheiten geeignet ist, wobei das Polypeptid aus 17 bis 70 Aminosäureresten zusammengesetzt ist und in seinem Mokekül die Aminosäuresequenz 180-196 des in Anspruch 1 genannten Polypeptids enthält.

**3.** Polypeptid nach Anspruch 2 mit der folgenden Aminosäuresequenz:

| | | | |
|---|---|---|---|
| 171 | 181 | 191 | 201 |
| GVITVEESNT | FGLQLELTEG | MRFDKGYISG | YFVTDPERQE |
| 211 | 221 | 231 | |
| AVLEDPYILL | VSSKVSTVKD | LLPLLEKVIG. | |

**4.** Verbindung nach Anspruch 2 oder 3, welche an mindestens ein Radikal gekuppelt ist, welches ihre Antigenizität und Immunogenizität verstärkt.

**5.** Verbindung nach Anspruch 4, wobei das die Immunogenizität verstärkende Radikal ein Radikal eines Peptids, des Tetanustoxoids, Diphterietoxoids, der $\beta$-Galactosidase oder eines mikrobiellen äußeren Membranproteins ist.

**6.** Multimere der Verbindungen nach Anspruch 2 oder 3.

**7.** Mikroorganismen, welche eine Verbindung nach Anspruch 2 oder 3 als solche oder als Teil eines Fusionsproteins oder als Multimer exprimieren.

**8.** Mikroorganismen nach Anspruch 7, welche gentechnologisch veränderte Vaccinia- oder Salmonella-Stämme sind.

**9.** Pharmazeutische Zusammensetzung, insbesondere impfstoff gegen Autoimmunkrankheiten, insbesondere arthritische Zustände, umfassend eine Verbindung nach einem der Anspüche 2 bis 6 oder einen Mikroorganismus nach Anspruch 7 oder 8.

**10.** Diagnostische Zusammensetzung, umfassend eine Verbindung nach einem der Ansprüche 2 bis 6.

**11.** Kit zur Durch führung immunologischer Tests, umfassend ein Behältnis mit einer Verbindung nach einem der Ansprüche 2 bis 6 oder mit der diagnostischen Zusammensetzung nach Anspruch 10.